# EUROPEAN PATENT APPLICATION

(11) **EP 3 238 720 A1**
(43) Date of publication of application: **01.11.2017**
(21) Application number: 16167694.5
(22) Date of filing: 29.04.2016
(51) Int. Cl.: A61K 31/21, A23K 20/10

(54) **ANTI-METHANOGENIC COMPOSITIONS AND USES THEREOF**

(71) Applicant: DSM IP Assets B.V., 6411 TE Heerlen (NL)
(72) Inventor: The designation of the inventor has not yet been filed
(74) Representative: Berg, Katja

(57) **Abstract**

The present invention relates to the use of specific nitrooxy compounds as methyl-coenzyme M reductase (MCR) inhibitor, in particular for inhibiting the methane production in Methanothermobacter wolfeii, Methanobrevibacter smithii, Methanobrevibacter millerae, Methanobacterium bryantii, as well as Methanosphaera stadtmanae.

## Description

The present invention relates to the use of specific nitrooxy compounds as methyl-coenzyme M reductase (MCR) inhibitor, in particular for inhibiting the methane production in Methanothermobacter wolfeii, Methanobrevibacter smithii, Methanobrevibacter millerae, Methanobacterium bryantii, as well as Methanosphaera stadtmanae.

Methane formation in methanogenic archaea such as methanobacteriacea is catalysed by methyl-coenzyme M reductase (MCR), involving methyl-coenzyme M and coenzyme B as substrates.

The family of methanobacteriaceae is represented by five genera. Examples thereof are Methanobacterium, Methanothermobacter, Methanobrevibacter, and Methanosphaera, each of which contains several species and consecutively various strains. Members of this family are very strict anaerobes and grow by oxidizing H₂ while producing methane (CH₄).

Surprisingly it has now been found that the activity of purified MCR was significantly reduced or even inactivated (does dependent) by specific nitrooxy compounds. Furthermore, it was surprisingly found that these compounds were particularly suitable for reducing/ inhibiting the growth as well as the methane production in specific methanobacteriacea which are Methanothermobacter wolfeii (M. wolfeii), Methanobrevibacter smithii (M. smithii), Methanobrevibacter millerae (M. millerae), Methanobacterium bryantii (M. bryantii), as well as Methanosphaera stadtmanae (M. stadtmanae).

Thus, in a first embodiment, the invention relates to a method of inhibiting MCR, preferably MCR-I, which method comprises contacting said MCR with an effective amount of at least one nitrooxy compound of formula (I), 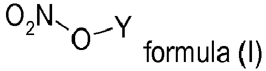 wherein
Y has the molecular formula CₐH_{b}O_{d}NₑS_{g} wherein
a is an integer between 1 and 25, preferably between 1 and 10
b is an integer between 2 and 51, preferably between 2 and 21
d is an integer between 0 and 10, preferably between 0 and 10
e is an integer between 0 and 5, preferably between 0 and 3
g is an integer between 0 and 3, preferably between 0 and 1,
or a salt thereof effective for inhibiting the enzyme.

In all embodiments the term 'an integer between X and 'Y is inclusive and also contains the X and the Y value.

The residue Y may be straight chained, branched, cyclic, bicyclic, aromatic as well as heteroaromatic and may contain one or more-OH, CN, -COOH, -NH₂, -ONO₂ group(s) and/ or one or more -NH-, -OC(=O)-, -(O=)CO-, -SO₂-, -C(=O)--, -C(=O)NH-, -O- or -N= structural elements at any position. Exemplary compounds of formula (I) are illustrated in table 1.

It is well understood to a person skilled in the art, that the nitrooxy group(s) in formula (I) are bound to one of the carbon atom(s) of Y.

In a preferred embodiment, the compounds of formula (I) contain 1 to 4 nitrooxy groups, preferably 1, 2 or 4 nitrooxy groups.

In all embodiments of the present invention, preferred compounds of formula (I) are compounds, wherein a is comprised between 1 and 10, preferably between 3 and 8.

In all embodiments of the present invention, particular preferred compounds of formula (I) are compounds, wherein g is 0 or 1.

In all embodiments of the present invention, it is to be understood that compounds of formula (I) can be in any isomeric form.

Preferred compounds of formula (I) according to the present invention are compounds of formula (II), 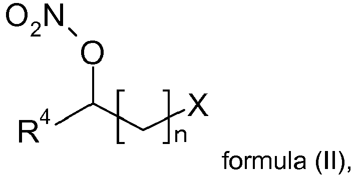 wherein
n is comprised between 0 and 12, preferably comprised between 0 and 6 and, wherein, if n ≠ 0, the carbon chain is a linear, a cyclic, or branched aliphatic carbon chain which may be non-substituted or substituted with up to 3 hydroxyl-, alkoxy-, amino-, alkylamino-, dialkylamino- or nitrooxy groups, or an alkenyl, or an alkynyl carbon chain mono- or polyunsaturated and in any isomeric form,
R⁴ is independently, hydrogen or a saturated straight, cyclic or branched chain of an alkyl or alkenyl group containing 1 to 12, preferably 1 to 6 carbon atoms,
X is hydrogen, R⁵, R⁵≡N, -OR⁵, -OCOR⁵, -NR⁵R⁶, -ONO₂,-COOR⁵, -CONR⁵R⁶, -NHSO₂R⁵, or -SO₂NHR⁵, preferably
R⁵ and R⁶ are independently, hydrogen, C₁-C₁₂ straight, branched or cyclic alkyl chain, non substituted or substituted with up to 3 hydroxyl-, alkoxy-, amino-, alkylamino-, dialkylamino- or nitrooxy groups, alkenyl, or alkynyl carbon chain which may be mono or polyunsaturated, and in any isomeric form.

It is to be understood in the above definition of compounds of formula (II) that when n > 2, the carbon chain can be linear or branched at any position along the carbon chain. In addition, the carbon chain can be branched by multiple branches at different positions along the carbon chain. Moreover, when n > 3, the aliphatic carbon chain may form a cyclic moiety. This cyclic moiety can carry the nitrooxy moiety at any position (2, 3, 4), and it can also be branched at multiple positions by any aliphatic groups. The branched aliphatic groups are preferably, methyl, ethyl or propyl. Moreover, the carbon chain may be further substituted with up to 3 hydroxyl-, alkoxy-, amino-, alkylamino-, dialkylamino- or nitrooxy groups.

In the above definition of derivatives of the formula (II) a preferred alkyl group is methyl, ethyl, propyl, isopropyl, butyl, sec. butyl, isobutyl, pentyl, neopentyl, hexyl, cyclohexyl, and 2-ethyl-hexyl and octyl. Furthermore any alkyl or alkenyl group containing three or more carbon atoms can be straight chain, branched, or cyclic. In addition for the straight chain or branched C₂-C₁₀-alkenylene group, this is understood to encompass alkenylene groups with one or (from C₄) more double bonds; examples of such alkenylene groups are those of the formulae -CH=CH-, -CH=CH-CH₂-, -CH=CH-(CH₂)₃- and -(CH=CH)₂-.

Particular advantageous nitrooxy compounds of formula (I) in all embodiments of the present invention are compounds of formula (III) 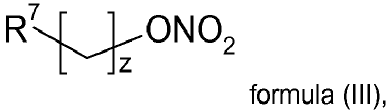 wherein
z is an integer between 1 and 10,
R⁷ is H, C₁-C₆ alkyl, phenyl, -OH, -NH₂, -CN, -COOH, -O(C=O)R⁸, -NHC(=O)R⁸, SO₂NHR⁸, or -ONO₂, and
R⁸ is C₁-C₆ alkyl, phenyl, pyridyl such as preferably 2-pyridyl
with the proviso that when z is > 3 the hydrocarbon chain may be interrupted by -O- or-NH-.

Particular preferred compounds of formula (I) in all embodiments of the present invention are compounds of formula (III) wherein
z is an integer between 3 and 9
R⁷ is OH, COOH or -ONO₂,
with the proviso that if z is 4 the hydrocarbon chain may be interrupted by -NH- such as in particular the compounds of formula (IV) R7-(CH₂)₂-NH-(CH₂)₂-ONO₂ (IV).

The term *'contacting'* comprises contacting a microorganism having MCR or contacting the (natural) environment, respectively habitat of such a microorganism with one or more nitrooxy compounds of formula (I) or a salt thereof.

Preferably said microorganism having MCR is a methanobacteriacea, such as in particular a Methanothermobacter, a Methanobrevibacter, a Methanobacterium or a Methanosphaera such as most in particular M. wolfeii, M. smithii, M. millerae, M. bryantii, as well M. stadtmanae.

In another embodiment the invention relates to a method for reducing or inhibiting the methane production in a methanobacteriacea selected from the group consisting of M. wolfeii, M. smithii, M. millerae, M. bryantii, and M. stadtmanae, said method comprising administering to a medium comprising at least one of said methanobacteriacea an effective amount of at least one nitrooxy compound of formula (I) or a salt thereof.

In a further embodiment, the present invention also relates to a method for reducing or inhibiting the growth of a methanobacteriacea selected from the group consisting of M. wolfeii, M. smithii, M. millerae, M. bryantii, and M. stadtmanae, said method comprising administering to a medium comprising at least one of said methanobacteriacea an effective amount of at least one nitrooxy compound of formula (I) or a salt thereof.

The term *'medium'* encompasses any medium in which the microorganism is present such as e.g. a culture medium comprising the isolated methanobacteriacea as well as an environment wherein the respective methanobacteriacea naturally occurs, i.e. its natural habitat such as e.g. the human gut (M. smithii, M. stadtmanae), the gut of termites (strains which are closely related to Methanobacterium bryantii) or landfills (M. bryanthii).

As the gut of termites contains as main methanogens three strains which are closely related to M. bryantii (>99% nucleotide identity) [see e.g. P. Deevong et al: Microbes and Environments 29(3), 221-226 (2004)], the organic molecules substituted at any position with at least one nitrooxy group, as defined by formula (I) or a salt thereof are also particularly suitable to reduce methane emission from termite colonies.

Thus in further embodiment the invention also relates to a method to reduce methane emissions from termite colonies, said method comprising applying an effective amount of at least one nitrooxy compound of formula (I), or a salt thereof (with all the preferences and definitions given herein) to a termite colony, in particular to the surface thereof.

M. bryantii has a high abundance in landfill sites (also known as a tip, dump, rubbish dump, garbage dump or dumping ground and historically as a midden).
Thus in another embodiment, the present invention also relates to a method of reducing the production of methane emanating landfills, said method comprising applying an effective amount of at least one nitrooxy compound of formula (I), or a salt thereof to the surface of such landfill sites.

In all embodiments of the present invention particularly preferred methanobacteriacea are selected from the group consisting of M. wolfeii, M. smithii, M. millerae, M. bryantii, and M. stadtmanae, such as most preferably from M. wolfeii, M. smithii, M. millerae and M. bryantii as these are affected already by very low concentrations of the nitrooxy compound of formula (I), or a salt thereof.

In another embodiment, the present invention relates to the use of at least one nitrooxy compound of formula (I), or a salt thereof as MCR-inhibitor.

In all embodiments of the present invention it is particular advantageous to select the nitrooxy compounds from the group consisting of 3-nitrooxypropanol, 9-nitrooxynonanol, 2-aminoethyl nitrate, 3-aminopropyl nitrate, 5-nitroxy pentanoic acid, 6-nitroxy hexanoic acid, bis-(2-nitrooxyethyl) ether, Bis(2-hydroxyethyl)amine dinitrate, 1,3-bis-nitrooxypropane, 1,4-bis-nitrooxybutane, 1,5-bis-nitrooxypentane, 3-nitrooxy-propyl hexanoate, 3-nitrooxy-propyl 5-nitrooxy-hexanoate, 2-(hydroxymethyl)-2-(nitrooxymethyl)-1,3-propanediol, 3-bis(nitryloxy)-2,2-bis(nitryloxy-methyl)-propan, N-ethyl-3-nitrooxy-propionic sulfonyl amide, 5-nitrooxy-pentanenitrile, 5-nitrooxy-pentane, 3-nitrooxy-propyl propionate, 3-nitrooxy-propyl benzoate, rac-4-phenylbutane-1,2-diyl dinitrate, benzylnitrate, N-[2-(nitrooxy)ethyl]-3-pyridinecarboxamide, 1,4:3,6-dianhydro-2,5-di-O-nitro-D-glucitol, 8-nitrooxy-2,6-dioxabicyclo[3.3.0]octan-4-ol and 2-nitro-5-nitrooxymethyl-furan, as outlined in table 1.

**Table 1: Particular advantageous nitrooxy compound for the purposes of the present invention**

| **#** | **Molecular structure** | **Chemical name** |
|---|---|---|
| 1 | 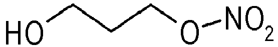 | 3-Nitrooxypropanol |
| 2 | 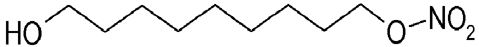 | 9-Nitrooxynonanol |
| 3 | 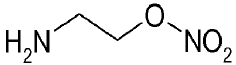 | 2-aminoethyl nitrate |
| 4 | 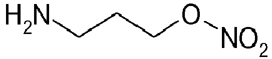 | 3-aminopropyl nitrate |
| 5 | 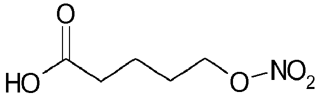 | 5-nitroxy pentanoic acid |
| 6 | 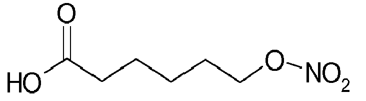 | 6-nitroxy hexanoic acid |
| 7 | 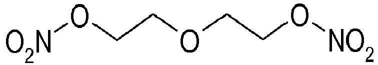 | Bis-(2-nitrooxyethyl) ether |
| 8 | 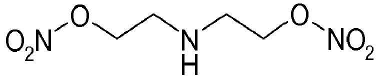 | Bis(2-hydroxyethyl)amine dinitrate |
| 9 | 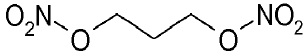 | 1,3-bis-Nitrooxypropane |
| 10 | 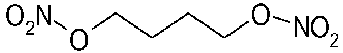 | 1,4-bis-Nitrooxybutane |
| 11 | 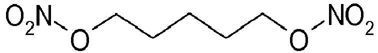 | 1,5-bis-Nitrooxypentane |
| 12 | 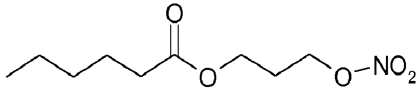 | 3-Nitrooxy-propyl hexanoate |
| 13 | 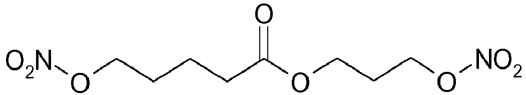 | 3-N itrooxy-propyl 5-nitrooxy-hexanoate |
| 14 | 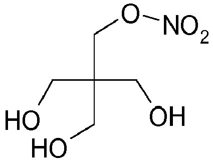 | 2-(Hydroxymethyl)-2-(nitrooxymethyl)-1,3-propanediol |
| 15 | 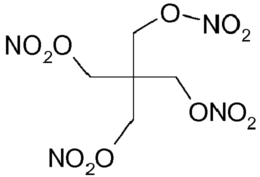 | 1,3-Bis(nitryloxy)-2,2-bis(nitryloxy-methyl)-propan |
| 16 | 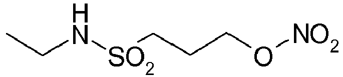 | N-Ethyl-3-n itrooxy-propion ic sulfonyl amide |
| 17 | 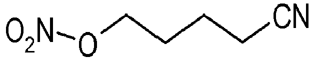 | 5-Nitrooxy-pentanenitrile |
| 18 | 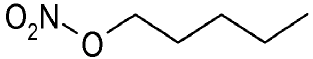 | 5-Nitrooxy-pentane |
| 19 | 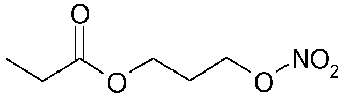 | 3-Nitrooxy-propyl propionate |
| 20 | 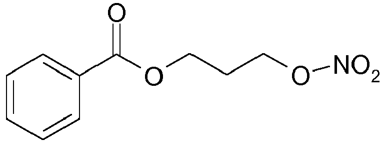 | 3-Nitrooxy-propyl benzoate |
| 21 | 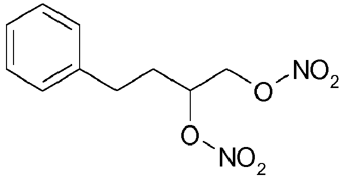 | rac-4-Phenylbutane-1,2-diyl dinitrate |
| 22 | 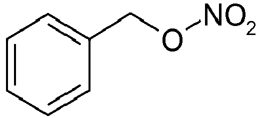 | Benzylnitrate |
| 23 | 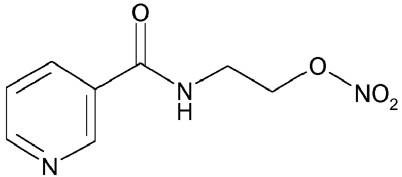 | N-[2-(Nitrooxy)ethyl]-3-pyridinecarboxamide |
| 24 | 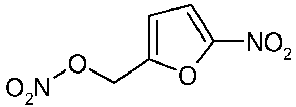 | 2-Nitro-5-n itrooxymethyl-furan |
| 25 | 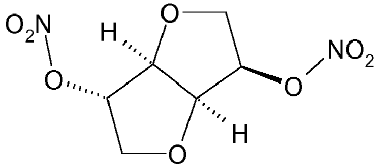 | 1,4:3,6-dianhydro-2,5-di-O-nitro-D-glucitol |
| 27 | 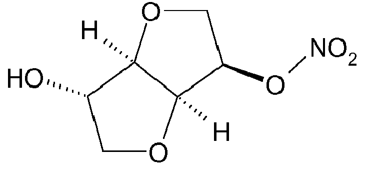 | 8-nitrooxy-2,6-dioxabicyclo[3.3.0]octan-4-ol |

Most preferred in all embodiments of the present invention is the use of 3-Nitrooxypropanol (CAS-No: 100502-66-7), 9-Nitrooxynonanol, 5-nitroxy pentanoic acid (CAS 74754-56-6), 6-nitroxy hexanoic acid (CAS 74754-55-5), Bis(2-hydroxyethyl)amine dinitrate (CAS 20830-49-3), 1,4-bis-Nitrooxybutane (CAS 3457-91-8) and 1,5-bis-Nitrooxypentane (CAS 3457-92-9)

The compounds according to the present invention are known and either commercially available or can be prepared in analogy to the processes as e.g. disclosed in WO2012/084629.

If the compounds of formula (I) can be converted into a salt (e.g. as they carry a -NH₂,-NH- or an acid group), this can be done according to a conventional method in the art, for example, by treating the respective compound with an organic or inorganic acid or a base in a solvent such as ethers, lower alcohols, ethyl acetate, hexane or the like at approximately room temperature. Preferred salts are e.g. the HNO₃⁻ salts.

In all embodiments of the present invention preferably only one nitrooxy compound of formula (I), or a salt thereof is used/ administered.

The term *'effective amount* as used herein refers to an amount necessary to obtain the desired physiological effect, i.e. a reduction of the enzyme activity or the methane emission of at least 50%, preferably at least 70% most preferably at least 90%. The physiological effect may be achieved by a single dose or by repeated doses. The dosage administered may, of course, vary depending upon known factors such as the specific characteristics of the particular medium or environment as well as its mode and route of administration; and can be adjusted by a person skilled in the art.

Generally, the term *'effective amount* as used herein refers to amounts of at least 0.01 µM, preferably to amounts in the range of 0.1 µM to 50 µM most preferably to amounts in the range of 0.1 µM to 30 µM based on the medium or environment comprising the enzyme or the methanobacteriaceae to be treated.

The invention will now be elucidated by way of the following examples, without however being limited thereto.

### Example 1: Inhibition of MCR by 3-Nitrooxypropanol (3-NOP)

### 1.1 Purified MCR

MCR (MCR-I) was purified from *M. marburgensis* grown exponentially in a 12 liter fermenter containing 10 liter culture gassed with 80% H₂/20% CO₂/0.01% H₂S at a rate of 10 liter per min and stirred at a rate of 1000 rpm. The medium used was that of Schönheit et al. (Arch Microbiol 127(1):59-65 (1980)). When the culture had reached an OD of approximately 4, gassing was switched to 100% H₂ and the culture was cooled to 4°C and harvested yielding about 80 g cells (wet mass). From the cells MCR I was anaerobically purified in the presence of coenzyme M. The protein concentration was determined by measuring the absorbance of oxidized enzyme (MCRₛᵢₗₑₙₜ) at 420 nm using ε = 44,000 M-¹cm⁻¹ for a molecular mass of 280,000 Da.

### 1.2 MCR activity assay

### 1.2.1 Reduction of methane formation

The assay of methane formation from methyl-coenzyme M and coenzyme B was performed in closed 7.5 mL bottle containing 400 µL assay solution and 100% N2 as gas phase. The solution contained 50 mM Tris-HCl (pH 7.6), 10 mM methyl-coenzyme M, 1 mM coenzyme B, 10 mM dithiothreitol (to regenerate coenzyme B from CoM-S-S-CoB) and purified MCR. Dithiothreitol was used as an electron source to regenerate coenzyme B. The reaction was started by the addition of enzyme. After 0, 3, 6 and 9 min, 100 µL gas samples were withdrawn from the headspace and analyzed for methane gas chromatographically with flame ionization detection (Buck Scientific, model 910).

**Table 2: Results**

| | Methane concentration [µM] | | |
|---|---|---|---|
| | 3min | 6min | 9min |
| No inhibitor | 0.095 | 0.132 | 0.163 |
| 0.1 µM 3-NOP | 0.068 | 0.082 | 0.086 |
| Methane reduction | -28% | -38% | -47% |

As can be retrieved, already 0.1 µM 3-NOP quickly reduces the methane concentration and thus the MCR activity by about 50%.

### 1.2.2 EPR signal determination

MCR purified as described above was supplemented with 3- NOP to a final concentration of 0.5 µM, 5 µM, 30 µM, 40 µM, 60 µM, 80 µM and 100 µM and incubated for 15 min at room temperature in an EPR tube that was subsequently frozen in liquid nitrogen. Continuous-wave (CW) electron paramagnetic resonance (EPR) spectra were measured at X-band (9-GHz) frequency on a Bruker EMX spectrometer fitted with an ER-4119-HS (high-sensitivity) perpendicular-mode cavity. Measurements at 77 K were performed by fitting the cavity with a liquid nitrogen finger Dewar. All spectra were recorded with a field modulation frequency of 100 kHz, modulation amplitude of 0.6 mT, and frequency of 9.386 GHz. The EPR signal intensities were determined by measuring the respective EPR-active species under non-saturating conditions. Since the signals represent the first derivative of the absorption-type signal, the spectra were double integrated and the surface area of each signal, corrected for the presence of two nickel sites per MCR, was compared with that of a 10 mM copper perchlorate standard (10 mM CuSO4; 2 M NaClO₄; 10 mM HCl). The values obtained this way were compared to the known enzyme concentration, which was set to 100%. When more than one signal is present, each signal is simulated, the compound spectrum is reproduced, and the double-integration value of each individual component is obtained. The BioEPR package developed by Fred Hagen was used for spectral simulation and double integration of the signals. Only the intensities of the paramagnetic species (red1 and ox1) could be determined as described. The amount of the EPR-inactive form, MCRₛᵢₗₑₙₜ, was assigned as the difference between the concentration of MCR present and the concentration of the paramagnetic species.

It was found that already at a concentration of 10 µM of 3-NOP the MCR activity was reduced by 50 %, whereas a concentration of > 30 µM of 3-NOP led to almost complete inhibition of the enzyme

### Example 2: Influence of 3-Nitrooxypropanol (3-NOP) on the growth/ methane production in selected archaea

The methanogens outlined in table 3 were grown in 5 mL of the respective culture medium at 37°C in 15-mL Hungate tubes sealed with butyl rubber septum and aluminum seals. The 8 mL gas phase was 80% H₂/20% CO₂ at 2 x 10⁵ Pa pressure. In the case of *M. stadtmanae* and *M. barkeri* H₂ and methanol (100 mM) was the energy source.

Methanogens growth was followed by CH₄ production using a flame ionization-detector GC (0.5 ml of gas from headspace, manual injection in the GC (HP Hewlett 5890, Packard Series II, Waldbronn, Germany) using a 1 mL Sample-Lock® syringe (Hamilton, Nevada, USA). The concentration of CH₄ was determined using a standard curve generated by injecting different volumes of 99.9 % pure CH₄ prior to and after the injection of samples.
Three consecutive 5 mL batches of incubations were conducted. In each batch the following treatments were used in triplicate: (i) blanks, only media; (ii) controls, cultures in the absence of inhibitor; (iii) 3-NOP added to a final concentration of 0.5 µM, 5 µM, 50 µM, 100 µM, 250 µM and 500 µM. 3-NOP was added on day 2 after culture inoculation with 0.5 mL of an actively growing culture. The growth was measured over 12 days every 2 days coinciding with new pressurization of headspace to 2 bar with 80:20 H₂:CO₂ gas mixture.

Results Table 4 shows the lowest concentration of 3-NOP able to significantly decrease the growth/ methane production (>50%) in the respective archaea already after 2 days, which decrease was also maintained over the whole measurement period of 12 days.

**Table 3: Methanogens**

| Strain | Methanogens | Culture medium | Source* |
|---|---|---|---|
| 861 | *Methanobrevibacter smithii* | 119 | DSMZ |
| 2970 | Methanothermobacter wolfeii | Schönheit et al⁺ | DSMZ |
| 16643 | *Methanobrevibacter millerae* | 119 | DSMZ |
| 863 | *Methanobacterium bryantii* | 119 | DSMZ |
| 3091 | *Methanosphaera stadtmanae* | 322 | DSMZ |
| 1539 | *Methanomicrobium mobile* | 161 | DSMZ |
| 800 | *Methanosarcina barkeri* | 120 | DSMZ |

| | | | |
|---|---|---|---|
| *Deutsche Sammlung von Mikroorganismen und Zellkulturen, Braunschweig, Germany ⁺P. Schönheit et al, Arch Microbiol 127(1):59-65 (1980) | | | |

**Table 4 Results**

| **#** | **Archaea** | **Concentration 3-NOP** |
|---|---|---|
| *Inv-1* | *Methanothermobacter wolfeii* | < 1 µM |
| *Inv-2* | *Methanobrevibacter smithii* | 2.5 µM |
| *Inv-3* | *Methanobrevibacter millerae* | 2.5 µM |
| *Inv-4* | *Methanobacterium bryantii* | 1 µM |
| *Inv-5* | *Methanosphaera stadtmanae* | 5 µM |
| *Ref*-*1* | *Methanomicrobium mobile* | 50 µM |
| *Ref-2* | *Methanosarcina barkeri* | 50 µM |

## Claims

1. A method of inhibiting MCR, which method comprises contacting MCR with an effective amount of at least one nitrooxy compound of formula (I), 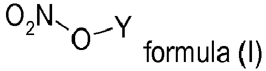 wherein Y has the molecular formula CₐH_{b}O_{d}NₑS_{g}
wherein
a is an integer between 1 and 25, preferably between 1 and 10
b is an integer between 2 and 51, preferably between 2 and 21
d is an integer between 0 and 10, preferably between 0 and 10
e is an integer between 0 and 5, preferably between 0 and 3
g is an integer between 0 and 3, preferably between 0 and 1,
or a salt thereof effective for inhibiting the enzyme.

2. The method according to claim 1, wherein the at least one nitrooxy compound of formula (I) is selected from the group consisting of 3-nitrooxypropanol, 9-nitrooxynonanol, 2-aminoethyl nitrate, 3-aminopropyl nitrate, 5-nitroxy pentanoic acid, 6-nitroxy hexanoic acid, bis-(2-nitrooxyethyl) ether, Bis(2-hydroxyethyl)amine dinitrate, 1,3-bis-nitrooxypropane, 1,4-bis-nitrooxybutane, 1,5-bis-nitrooxypentane, 3-nitrooxy-propyl hexanoate, 3-nitrooxy-propyl 5-nitrooxy-hexanoate, 2-(hydroxymethyl)-2-(nitrooxymethyl)-1,3-propanediol, 3-bis(nitryloxy)-2,2-bis(nitryloxy-methyl)-propan, N-ethyl-3-nitrooxy-propionic sulfonyl amide, 5-nitrooxy-pentanenitrile, 5-nitrooxy-pentane, 3-nitrooxy-propyl propionate, 3-nitrooxy-propyl benzoate, rac-4-phenylbutane-1,2-diyl dinitrate, benzylnitrate, N [2-(nitrooxy)ethyl]-3-pyridinecarboxamide, 1,4:3,6-dianhydro-2,5-di-O-nitro-D-glucitol, 8-nitrooxy-2,6-dioxabicyclo[3.3.0]octan-4-ol and 2-nitro-5-nitrooxymethyl-furan.

3. The method according to claim 1, wherein the at least one nitrooxy compound of formula (I) is selected from the group consisting of 3-Nitrooxypropanol, 9-Nitrooxynonanol, 5-nitroxy pentanoic acid, 6-nitroxy hexanoic acid, Bis(2-hydroxyethyl)amine dinitrate, 1,4-bis-Nitrooxybutane and 1,5-bis-Nitrooxypentane.

4. The method according to anyone of claims 1 to 3, wherein contacting comprises contacting a microorganism having MCR or contacting the (natural) environment a microorganism having MCR with at least one nitrooxy compound of formula (I), or a salt thereof.

5. The method according to claim 4, wherein the microorganism is a methanobacteriaceae.

6. The method of claim 5, wherein the methanobacteriacaea is selected from the group consisting of M. wolfeii, M. smithii, M. millerae, M. bryantii, and M. stadtmanae" preferably from the group consisting of M. wolfeii, M. smithii, M. millerae and M. bryantii.

7. A method for reducing and/ or inhibiting the methane production in a methoanobacteriacea selected from the group consisting of M. wolfeii, M. smithii, M. millerae, M. bryantii, and M. stadtmanae, preferably from the group consisting of M. wolfeii, M. smithii, M. millerae and M. bryantii, the method comprising contacting a medium comprising said methoanobacteriacea with an effective amount of at least one nitrooxy compound of formula (I), or a salt thereof as defined in any one of claims 1 to

8. A method for reducing and/ or inhibiting the growth of a methoanobacteriacea selected from the group consisting of M. wolfeii, M. smithii, M. millerae, M. bryantii, and M. stadtmanae, preferably from the group consisting of M. wolfeii, M. smithii, M. millerae, and M. bryantii, the method comprising contacting a medium comprising said methoanobacteriacea with an effective amount of at least one nitrooxy compound of formula (I), or a salt thereof as defined in any one of claims 1 to 3.

9. A method to reduce methane emissions from termite colonies, said method comprising applying an effective amount of at least one nitrooxy compound of formula (I), or a salt thereof as defined in any one of claims 1 to 3 to a termite colony, in particular to the surface thereof.

10. A method of reducing the production of methane emanating landfills, said method comprising applying an effective amount of at least one nitrooxy compound of formula (I), or a salt thereof as defined in any one of claims 1 to 3 to the surface of such landfill sites.

11. The method according to any one of claims 1 to 10, wherein the effective amount is selected to achieve a a reduction of the enzyme activity respectively the methane emission of at least 50%, preferably of at least 70% most preferably of at least 90%.

12. The method according to any one of claims 1 to 11, wherein the effective amount is selected in the range of 0.1 µM to 50 µM, preferably in the range of 0.1 µM to 30 µM based on the medium or environment comprising the enzyme or the methanobacteriaceae to be treated.

13. Use of of at least one nitrooxy compound of formula (I), or a salt thereof as defined in any one of claims 1 to 3 as MCR-inhibitor.
